**Europäisches Patentamt**

**European Patent Office**

**Office européen des brevets**

(19)

(11) Publication number : **0 290 569 B1**

# EUROPEAN PATENT SPECIFICATION

(12)

(45) Date of publication of patent specification :
**17.06.92 Bulletin 92/25**

(51) Int. Cl.⁵ : **C12N 9/58,** C11D 3/386, C11D 7/42, C12R 1/79

(21) Application number : **87907943.2**

(22) Date of filing : **25.11.87**

(86) International application number :
**PCT/DK87/00146**

(87) International publication number :
**WO 88/03948 02.06.88 Gazette 88/12**

(54) **LOW-TEMPERATURE ACTIVE AKALINE PROTEASE FROM PAECILOMYCES MARQUANDII AND ITS PREPARATION.**

(30) Priority : **25.11.86 DK 5639/86**

(43) Date of publication of application :
**17.11.88 Bulletin 88/46**

(45) Publication of the grant of the patent :
**17.06.92 Bulletin 92/25**

(84) Designated Contracting States :
**AT BE CH DE FR GB IT LI LU NL SE**

(56) References cited :
**SE-B- 0 360 675
SE-B- 0 409 335
US-A- 3 652 399
US-A- 3 661 715
US-A- 3 723 250
US-A- 4 511 490
Chemical Abstracts, vol. 95, (1981), abstract no. 22917t, Rev. Roum Biochin 1980. 17(3), 209-15**

(73) Proprietor : **NOVO NORDISK A/S
Novo Allé
DK-2880 Bagsvaerd (DK)**

(72) Inventor : **BECK, Carol, Marie
20 Huntington
Bethel, CT 06801 (US)**
Inventor : **STROBEL, Robert, Joseph, Jr.
19 Ole Musket Lane
Danbury, CT 06810 (US)**

(74) Representative : **Brown, John David et al
FORRESTER & BOEHMERT
Franz-Joseph-Strasse 38
W-8000 München 40 (DE)**

## Description

The present invention is directed to a novel alkaline protease, to a method for the preparation thereof, and to its use in combination with other proteases. This protease may be advantageously employed as an additive for cleaning agents. Typical cleaning agents are detergent compositions for laundering.

## BACKGROUND OF THE INVENTION

Proteolytic enzymes produced by cultivation of microorganism strains from the genus Bacillus in suitable nutrient media are widely used in detergent compositions. Examples of such commercially available proteinase products are ALCALASE®, ESPERASE® and SAVINASE®, all supplied by NOVO INDUSTRI A/S, Denmark. These and the similar Bacillus derived enzyme products from other suppliers are enzymatically active in detergent solutions, at pH values in the range of from 8 to 11 and in the presence of the sequestering agents, surfactants and bleaching agents normally present in detergent solutions.

The protease in ALCALASE® is produced by cultivating strains of the species Bacillus licheniformis. The protease in ESPERASE® and SAVINASE® are obtainable by cultivation of alkalophilic Bacillus species according to U.S. patent 3,723,250, and are characterized by being of the serine type and showing optimal proteolytic activity at a pH value above about 9 and retaining 80 to 100 percent of maximum proteolytic activity at pH 12, said activities being measured against hemoglobin by the Anson method.

The temperature optimum of the above commercially available alkaline proteases is about 60°C. However, these commercial enzymes exhibit a relatively lower activity at room temperature.

## BRIEF STATEMENT OF THE INVENTION

This invention relates to the production, isolation, characterization and use of a novel alkaline protease obtained from a fungal microorganism. The protease producing microorganism is an isolate of Paecilomyces marquandii. This microorganism has not been previously known to produce a useful alkaline protease. The temperature optimum of this protease is about 45°C, some 15°C lower than that of the commercially available detergent enzymes. In addition, the fungal protease of this invention generates a protein hydrolysis pattern different from that generated by a Bacillus protease.

The fungal protease of this invention is more effective than the commercially available Bacillus proteases at low temperatures, e.g. in cool water, say 15°-25°C.

Its different hydrolysis pattern makes the fungal protease effective against some protein refractory to the action of the Bacillus proteases. In addition, synergism has been observed from mixtures made with the fungal protease from Paecilomyces marquandii and certain Bacillus proteases. Therefore, employment of the fungal protease in combination with a Bacillus protease is attractive and is contemplated by the inventors hereof.

## DISCUSSION OF THE INVENTION

Although many commercial uses exist for proteases, detergent usage represents a principal market, and appropriately, the detailed discussion of the Paecilomyces marquandii protease which follows is posed within the detergent usage utility context. It should, however, be appreciated that the many other uses for proteolytic enzymes which exist are contemplated by the inventors hereof.

It should be appreciated also that enzymes often are particularly sensitive to the conditions of usage, notably to pH, temperature and for detergent usage to presence of common sequestering agents, EDTA, for example. In consequence, a protease well adapted to usual American washing conditions for laundering cotton fabrics, which, for example, may be at pH 8-9 and at 60°C, will be less effective under the low temperature washing conditions recommended for laundering many modern-day fabrics. Thus, the lower temperature optimum of the Paecilomyces marquandii protease is a major advantage of this enzyme. In practical terms, the lower temperature optimum means that less of this enzyme is needed to generate a desired enzymatic activity level at 45°C in detergent solution (than, for example, would be required of a Bacillus protease now employed for detergent additive purposes). A related practical result is that detergent formulators can now provide an enzyme combination of the Paecilomyces marquandii protease with a Bacillus protease, e.g., ALCALASE®, ESPERASE® or SAVINASE®, that will be effective over the entire temperature range of cold water washing to (standard) hot water washing. Moreover, the combination of the Paecilomyces marquandii protease with certain Bacillus proteases produces a synergistic effect such that a mixture is more effective than either enzyme by itself. This is true at high as well as low dosages, as evidenced in Examples X and XI, respectively provided hereinafter A protease preparation comprising the alkaline protease from Paecilomyces marquandii exhibits about 40% greater capability for whitening protein-based stains on cotton (EMPA 116) under simulated laundering conditions at 15°C than the well known ALCALASE® protease.

## DETAILED DESCRIPTION OF THE INVENTION

According to a further aspect of this invention, there is provided a method for producing the novel alkaline protease, which process is characterized by cultivating a protease producing strain of Paecilomyces marquandii under aerobic conditions in a nutrient medium containing assimilable sources of carbon, nitrogen and phosphorus, followed by recovery of the protease preparation from the fermentation broth.

According to another mode of the invention, the protease from Paecilomyces marquandii is formulated into a mixed enzyme product comprising a Bacillus derived protease and the Paecilomyces marquandii protease. Preferred Bacillus derived proteases are ALCALASE®, ESPERASE® and SAVINASE®.

For further understanding of this invention, reference is made to the attached drawings wherein:

Figure 1 shows the elution chromatogram of digestion products obtained after treatment of oxidized insulin beta chain with the protease of Paecilomyces marquandii for two hours, and, for comparison, the digestion products obtained after treatment with the known proteases, ALCALASE® and SAVINASE®:

Figure 2 is a graphical presentation of the activity of protease of Paecilomyces marquandii at various temperatures and pH values, in the absence or presence of phenylmethysulfonyl fluoride (PMSF, 1 mM) or EDTA (10 mM).

Figure 3 is a graphical presentation of a washing study made with ALCALASE® alone and together with some of the Paecilomyces marquandii protease, at a relatively high dosage.

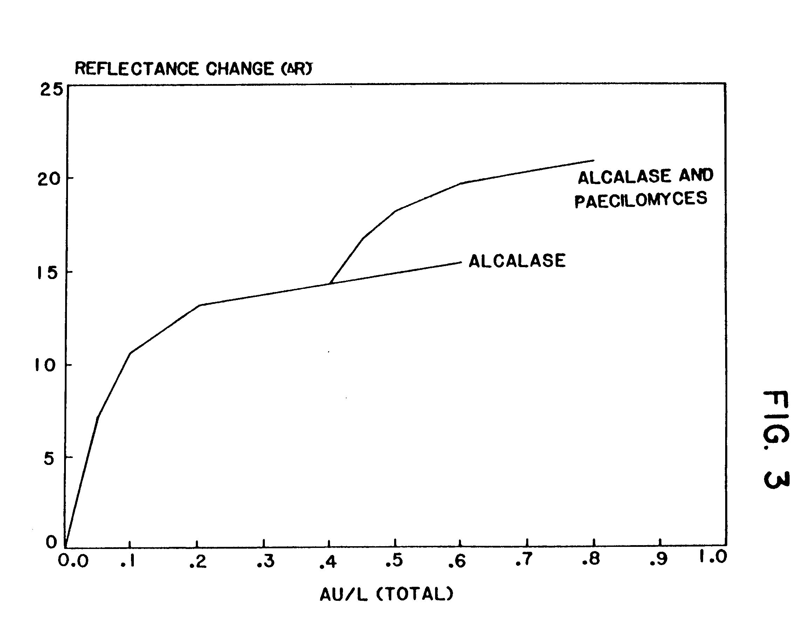

The reflectance change data graphed as Figure 3 evidences that combining the protease of Paecilomyces marquandii with a Bacillus protease results in an improved washing effect. Increasing dosage of ALCALASE® alone from 0.4 to 0.6 AU/l causes little improvement in the reflectance change. Employing an enzyme mixture of the same total proteolytic activity, e.g., 0.6 AU/l, generates substantially improved reflectance change.

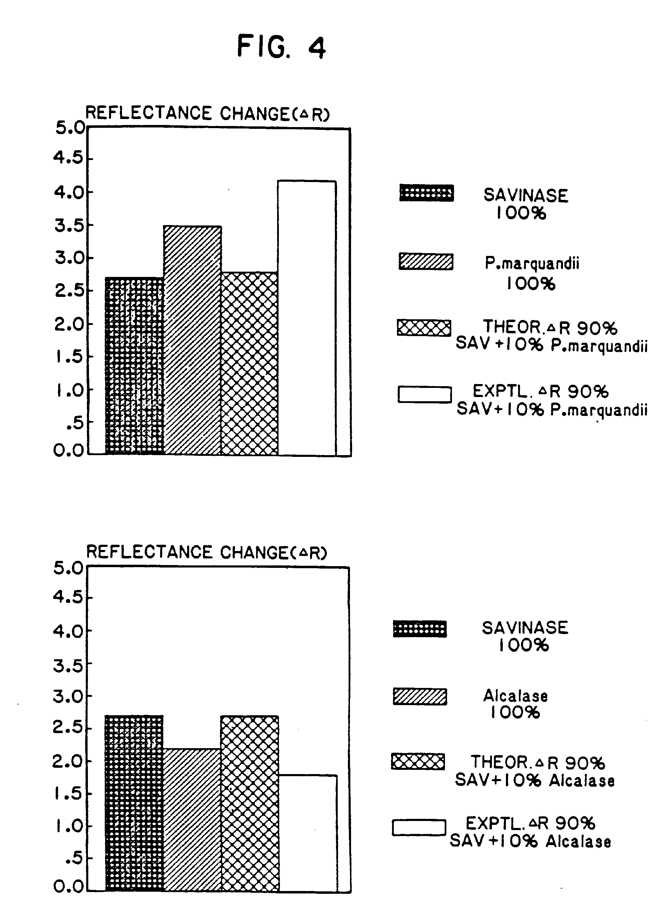

Figure 4 is a graphical presentation of the synergistic effect obtained in wash tests with a mixture of the fungal protease from Paecilomyces marquandii and another bacterial protease, SAVINASE®. The total enzyme dosage 0.1 KOU/liter is similar to that obtained by using commercial protease-containing detergents. In the top graph, the first two bars represent the reflectance change obtained for SAVINASE® and Paecilomyces marquandii alone, respectively. The third bar represents the (theoretical) Δ R value that can be expected for the 90:10 combination of SAVINASE® and Paecilomyces marquandii protease, i.e., 90% of the Δ R for SAVINASE® alone plus 10% of the Δ R for Paecilomyces marquandii alone. The fourth bar represents the experimental Δ R value obtained when such a 90:10 mixture was used in a wash. The observed Δ R value is 50% greater than the theoretical value, indicating thereby that synergism has occured.

For comparison, a mixture of two bacterial proteases was also tested, SAVINASE® and ALCALASE®. The results are illustrated in the lower half of Figure 4. In this case, the experimental Δ R obtained for the 90:10 mixture of SAVINASE® and ALCALASE® was less than that predicted, i.e., no synergy.

### The Microorganism

The microorganism of this invention is an aerobic, fungal isolate of Paecilomyces marquandii

Optimal temperature for growth: 20°C to 30°C, no growth at or above 35°C.

Optimal pH for growth: 7.5 - 10.

Gray-white colonies on Czapek Dox Agar slants; abundant spore formation responsible for pinkish-gray color of mature colonies.

Some degree of strain specificity may exist in the instance of the Paecilomyces marquandii. The isolate which elaborates the enzyme of this invention is NRRL 18048. Insofar as the inventors hereof have been able to ascertain, no strain of Paecilomyces marquandii has been identified as the type strain. The isolate that most nearly corresponds to a type strain appears to be NRRL 901 (same as ATCC 10525) and this strain fails to elaborate the protease of this invention. It is believed that NRRL 18048 and NRRL 901 are different variants of Paecilomyces marquandii.

### Assay for proteolytic activity

The proteolytic activity in Paecilomyces marquandii cultures was determined by the well known Anson hemoglobin method, cfr. Journal of General Physiology, 22, 79-89 (1959). One Anson unit is the amount of proteolytic enzyme digesting hemoglobin at pH value of 9.0 and a temperature of 25°C during a reaction time of 10 minutes with such an initial velocity that per minute there is formed such an amount of split products which cannot be precipitated with trichloracetic acid that these split products give the same color with phenol reagent as does one milliequivalent of tyrosine.

Proteolytic activity was also determined by the hydrolysis of casein and subsequent reaction of TCA-soluble peptides with o-phthaldialdehyde and beta-mercaptoethanol. The absorbance of the resultant complex is measured at 340 nm and compared to a serine standard. Reaction mixtures are comprised of 3 ml of 0.8% (w/v) Hammerstein casein and 0.5 ml of an appropriate enzyme dilution both in Universal Buffer 1 of Britton and Robinson, pH 9.5 (J. Chem. Soc. 1931, p. 1451). Mixtures are incubated for 30 minutes at 25°C, then the reaction terminated by addition of 1.25 mls stop reagent (3.6% w/v TCA, 6.0% w/v sodium acetate, and 3.78% v/v glacial acetic acid). In control reactions, the stop reagent is added prior to enzyme addition. After 20 minutes at 25°C, the reaction mixtures are filtered through Whatman® filter paper no. 42 or centrifuged.

An aliquot (200 $\mu$l) of the filtrate is added to 3 ml of OPA reagent containing 0.05M sodium tetraborate, 1% w/v sodium dodecylsulfate, 0.8 mg/ml o-phthaldialdehyde (OPA) (originally dissolved as a 40 mg/ml solution in ethanol), and 0.2% v/v beta-mercaptoethanol. After 2 minutes, the absorbance at 340 nm is determined. Similarly 200 $\mu$l aliquot of a serine standard (0.2 mg/ml) is added to 3 ml of OPA reagent and the $A_{340}$ determined. Activity is expressed in KOU (kilo OPA units) where 1 KOU is defined as the amount of proteolytic enzyme which results in the release of 1 mmol -$NH_2$ released per minute under the defined conditions.

## Preparation of Protease concentrate

The Paecilomyces marquandii strain NRRL 18048 is cultivated under aerobic conditions in a nutrient medium containing assimilable carbon and nitrogen together with other essential nutrients, the medium being composed in accordance with the principles of the known art.

Suitable carbon sources are carbohydrates, such as sucrose, glucose and maltose, or carbohydrate containing materials such as cereal grains, malt, rice and sorghum. The carbohydrate concentration incorporated in the medium may vary widely, e.g., 1 to 15%, but usually 8-10% will be suitable, the percentage being calculated as equivalents of glucose.

The nitrogen source in the nutrient medium should be of an organic nature. Among the organic nitrogen sources, quite a number are regularly used in fermentation processes involving the cultivation of fungi. Illustrative examples are soybean meal, cotton seed meal, peanut meal, casein, corn steep liquor, yeast extract, and albumin. In addition, the nutrient medium should also contain the usual trace substances.

Since Paecilomyces marquandii is phsychrophilic in that it is unable to grow at temperatures above 35°C, the cultivation is preferably conducted in the temperature range of 20°C to 30°C and at alkaline pH values. The alkaline pH may be obtained by addition of suitable buffers, such as sodium carbonate or mixtures of sodium carbonate and sodium carbonate or mixtures of sodium carbonate and sodium bicarbonate (after sterilization of the growth medium). For cultivation in tank fermentors, it is necessary to use artificial aeration. The rate of aeration may be that employed in conventional tank fermentation.

After fermentation, a liquid enzyme product may be produced by removal of coarse material from the broth and, if desired, through concentration of the broth by evaporation at low temperature of by reverse osmosis. Finally, preservatives and/or enzyme stabilizers such as propylene glycol may be added to the concentrate. A liquid product is suitable for incorporation in liquid detergents.

Solid enzyme preparations may be prepared from the purified and/or concentrated broth by precipitation with salts such as $Na_2SO_4$ or with water miscible solvents such as ethanol or acetone. Removal of the water in the broth by suitable drying methods such as spray drying may also be employed. The proteolytic activity of protease preparation so obtained is usually in the range of 0.1-2.0 AU/g.

Non-dusting granulates may be prepared by methods known in the art, e.g. according to GB patent 1,362,365 or US patent 4,106,991. Such granulates are suitable for incorporation in powder detergents.

## Peptide mapping

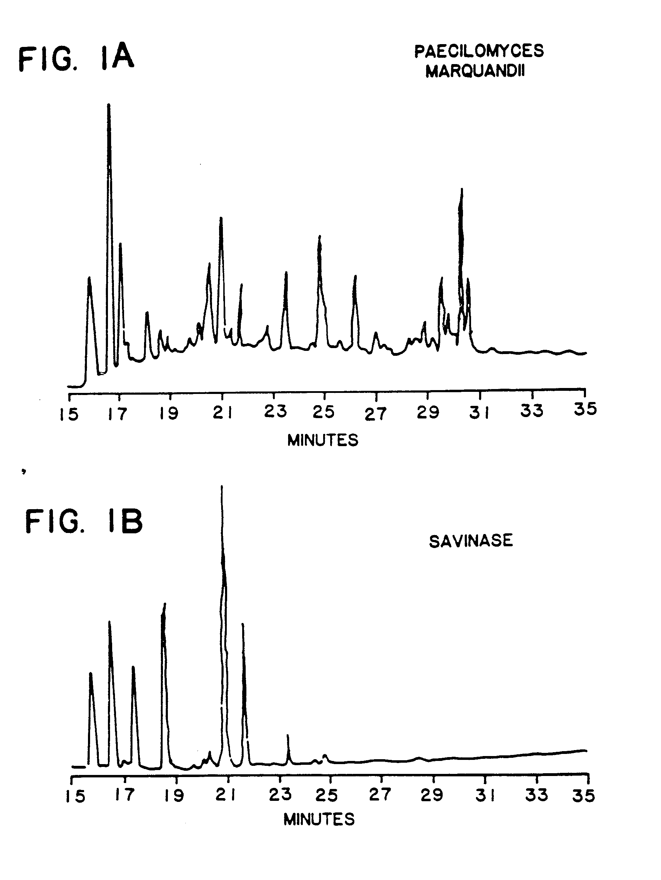

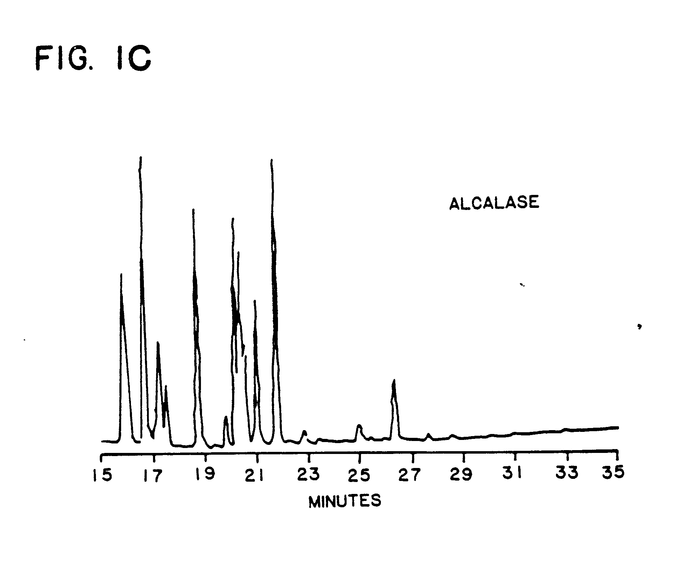

The peptides produced by protease-catalyzed digestion of the oxidized beta chain of insulin were separated by reverse phase HPLC. Chromatograms of peptide digestion products obtained through the action of the Paecilomyces marquandii protease, purified as described in Example VII, are shown in Figure 1. For comparison, the peptides generated by digestion with the known alkaline proteases, ALCALASE® and SAVINASE®, are also given. It may be seen that the protease of this invention is distinctly different from any of the Bacillus alkaline proteases with regard to its pattern of digestion of the oxidized insulin beta chain; a more random pattern of digestion is exhibited.

Immunochemical properties

Ouchterlony double immunodiffusion tests showed no cross reaction between the Paecilomyces marquandii and the alkaline serine proteases, ALCALASE® and SAVINASE® when antibodies prepared against these proteases were used as reference standards.

**EXAMPLE I**

Paecilomyces marquandii strain NRRL 18048 was cultivated at 26°C on a rotary shaking table (250 rpm) in 250 ml baffled Erlenmeyer flasks containing 25 ml of medium of the following composition:
Composition of the medium in gram per liter:

| | |
|---|---|
| Glucose | 100 |
| Soybean flour | 75 |
| Yeast extract | 1 |
| $Na_2HPO_4 \cdot 12H_2O$ | 10 |
| Pluronic® 25R2 | 0.1 |
| Sodium caseinate | 5 |

After sterilization, the pH of the medium was adjusted to 9.2 by addition of 5 ml of a 1M solution of sodium carbonate/sodium bicarbonate buffer.

After 7-9 days of incubation, the proteolytic activity of the culture was determined using the Anson metod as described above. The enzyme activity of the broth was 16-18 AU/1.

**EXAMPLE II**

Paecilomyces marquandii strains NRRL 18048 was cultivated in a medium consisting of the folloiwng (grams/liter):

| | |
|---|---|
| Maltodextrin | 20 |
| Soybean flour | 20 |
| $K_2HPO_4$ | 9 |
| $CaCO_3$ | 5 |
| Yeast extract | 2 |
| Pluronic® 25R2 | 1 |

After autoclaving, the pH was adjusted to 9.2 by addition of $NaCO_3/NaHCO_3$ buffer to a final concentration of 0.1M. The culture was incubated at 250 rpm, 30°C, for 24 hours. After this growth period, the culture (600 ml) was used to inoculate a fermentor containing 10 liters of the following medium (grams/liter):

| | |
|---|---|
| Glucose | 50 |
| Soybean flour | 100 |
| $K_2HPO_4$ | 5 |
| $CaCO_3$ | 5 |

After sterilization, the medium was adjusted to pH 8.2 with 2M $Na_2CO_3$. The pH fell to about 7.0 during the early stages of growth in the fermentor. The temperature was controlled at 30°C initially, and was lowered to 25°C when the pH began to rise above 7.0. From this point on, pH was controlled not to fall below 7.0 by addition of KOH or $Na_2CO_3$ and not to exceed 7.6 by addition of $H_3PO_4$. Plurronic® was used to control foam. Agitation and aeration were increased as $O_2$ demand increased; the fermentor was operated at maximum oxygen transfer rate by 24 hours. After 120 hours, protease titer was determined using the Anson assay method, a value of 25 Anson units/liter being indicated.

The culture broth was filtered to become the protease product employed in the following Examples.

**EXAMPLE III**

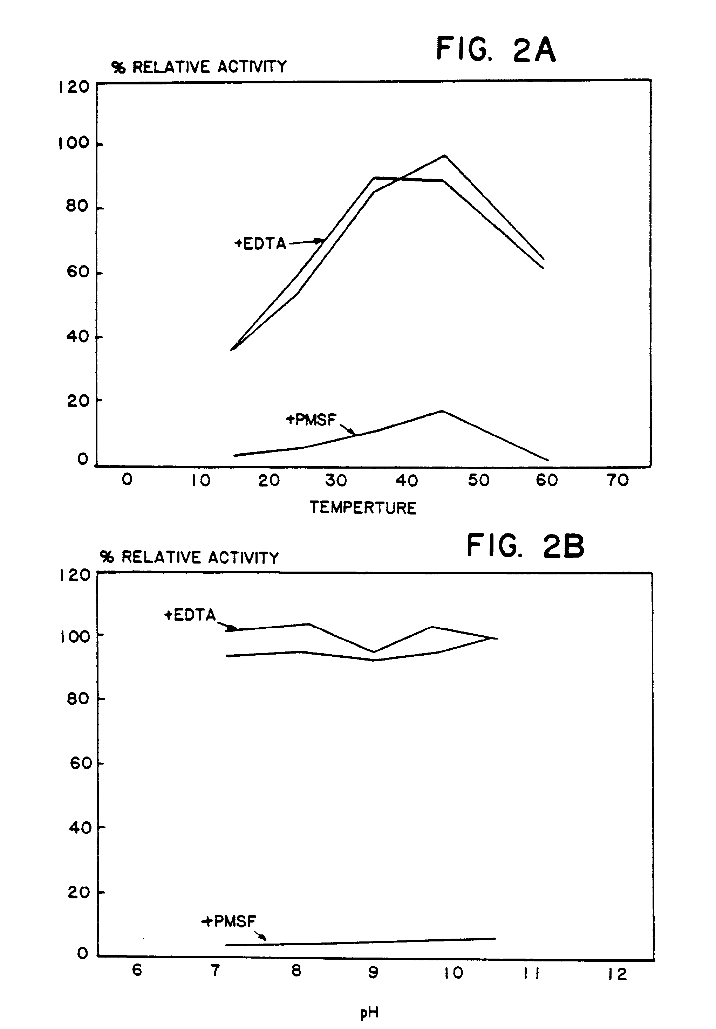

The activity of the protease from Example II as a function of pH and temperture was determined using casein as substrate according to the assay method described above. In addition, the effects of EDTA (10 mM) and phenylmethylsulfonyl fluoride (PMSF, 1 mM) on protease activity were examined. Results are depicted graphically in Figure 2. As seen in this figure, the temperature optimum is 45°C, at least 15°C lower than that of the commercial proteases ALCALASE® and SAVINASE®. The protease also shows close to maximal activity throughout the pH range 7-10.5. In addition, protease activity is almost completely inhibited in the presence of PMSF, not EDTA, indicating that the enzyme is a serine protease.

**EXAMPLE IV**

The stability of the protease of Example II in the presence of various detergent components at 25°C or 40°C was determined as follows:

Protease wad diluted to 0.5 AU/l in 0.01M borate buffer, pH 9.5 with or without the additional components shown in Table I. The samples were incubated at 25°C or 40°C for 30 minutes, then assayed at 25°C for protease activity as described previously. Results are shown in Table I.

The protease retains at least 83% of its activity at either 25°C or 40°C in any of the detergent components tested, making it suitable for laundering use.

## TABLE I

Stability of the protease from Paecilomyces marquandii in the presence of detergent.

| Components | Activity remaining (% of the 25°C buffer value) | |
| --- | --- | --- |
| | 25°C | 40°C |
| – | 100 | 103 |
| CaCl$_2$ (10 mM) | 105 | 99 |
| EDTA (10 mM) | 97 | 83 |
| Sodium tripolyphosphate (STPP, 0.1%) | – | 101 |
| "Tide" (no phosphate, 0.15%) | 104 | 89 |

**EXAMPLE V**

The ability of the protease to digest various proteinaceous substrates in the presence of detergent was tested and compared to the commercial protease, ALCALASE®. The substrates used were whole blood, casein, and grass.

Whole bovine blood, which was obtained as a spray dried powder, and casein (Hammerstein) were dissolved in 0.05M borate, pH buffer to a final concentration of 0.1% (w/v) in the presence of detergent (0.15% w/v, no phosphate) and water hardeners (150 ppm). The protease of Example II and, for comparison, ALCALASE® were added at the indicated activity level and reaction mixtures were incubated at 15°C for 15 minutes. Reactions were terminated by the addition of TCA and the number of TCA-soluble amino groups determined as described above using o-phthaldialdehyde.

A grass extract was prepared by homogenizing freshly cut grass (10 g) in distilled water (50 mls) for 5 minutes, then pressing through cheesecloth. The extract was thoroughly dialyzed into 50 mM borate, pH 10 buffer. Tide (no phosphate) and water hardeners were added directly to the extract to give concentrations of 0.15% and 150 ppm, respectively. The protease of Example II or ALCALASE® was added and the reactions carried out as described above, with the exception that the reaction conditions were 25°C and 30 minutes.

The degree to which the protease hydrolyzed each substrate relative to ALCALASE® is shown in Table II.

From the results in Table II, it is apparent that at the same dosage level, the Paecilomyces marquandii is more efficient than ALCALASE® in digesting blood, casein, and grass proteins in the presence of commonly used detergents.

TABLE II

Proteolytic digestion of blood, casein, and grass in the presence of detergent

| Substrate | Enzyme dose level (AU/l) | Degree of digestion (% of ALCALASE® value) |
|---|---|---|
| Whole blood | 0.1 | 116 |
| | 0.3 | 117 |
| Casein | 0.1 | 166 |
| | 0.3 | 134 |
| Grass | 0.1 | 265 |
| | 0.2 | 229 |

**EXAMPLE VI**

This example illustrates the effects of various commercial detergents on the ability of protease from Example II to digest blood protein. A comparison is also made to the commercial protease ALCALASE® in the same detergents.

Reaction mixtures contained denatured whole blood (0.1% w/v), 0.15% detergent, 150 ppm water hardness, 8.2 mM sodium borate, and 0.05 AU/l protease. Digestions were carried out for 15 minutes at 15°C, then the TCA-soluble reaction products measured as described previously. Results are shown in Table III.

## TABLE III

Proteolytic digestion of blood in the presence of five commercially available detergents drawn from the following list: tide, all, bold 3, Wisk, Dynamo, Era plus and Cheer.

| Detergent[a] | Protease | % digestion relative to ALCALASE® |
|---|---|---|
| 1 | P. marquandii | 129 |
|  | ALCALASE® | 100 |
| 2 | P. marquandii | 144 |
|  | ALCALASE® | 100 |
| 3 | P. marquandii | 133 |
|  | ALCALASE® | 100 |
| 4 | P. marquandii | 103 |
|  | ALCALASE® | 100 |
| 5 | P. marquandii | 154 |
|  | ALCALASE® | 100 |

a The pH of each reaction mixture was largely due to the detergent and ranged from pH 7.8-10.4

**EXAMPLE VII**

The protease prepared in Example II was purified as follows:

The culture broth was first dialyzed extensively into 25 mM sodium acetate, pH 5 buffer. Precipitated material formed during dialysis was removed by centrifugation. The dialyzed sample was loaded onto a carboxymethyl cellulose (CM-52) column which had been equilibrated in 25 mM sodium acetate, pH 5. Protease activity bound to the resin and was subsequently eluted in 25 mM sodium acetate, pH 5 containing 0.2M NaCl. Fractions containing protease activity were pooled and concentrated with an Amicon UM-2 membrane. The sample was then loaded onto a Sephadex G-100 column which had been equilibrated in 50 mM Tris-HCl, pH 7.0 buffer. The protease was eluted from the column using the same buffer, and active fractions were pooled. The protease sample was further purified on a hydroxyapatite column equiiibrated in 50 mM Tris-HCl, pH 7.0. In this buffer, protease adsorbed to the column, then was eluted with a gradient in which the ionic strength of Tris decreased and phosphate buffer increased. Protease activity was eluted at a phosphate buffer concentration of 10 mM, pH 7.0. The resultant specific activity of the protease was 47 Anson units/gram.

TABLE IV

Table IV summarizes the purification of protease from Example II

| Step | Protease activity (AU/l) | Volume (mls) | Protein Conc. (mg/ml) | Specific activity (AU/g) | Total recovery % | Fold purification |
|---|---|---|---|---|---|---|
| Dialyzed culture supernatant | 19.3 | 227 | 8.6 | 2.25 | 100 | 1 |
| CM-52 | 53.0 | 62.9 | 4.2 | 12.5 | 76 | 5.6 |
| Sephadex G-100 | 21.6 | 135 | 0.96 | 22.5 | 67 | 10 |
| Hydroxy-apatite | 20.1 | 105 | 0.43 | 46.8 | 48 | 21 |

## EXAMPLE VIII

This experiment was carried out to determine the specific activity of the purified protease.

Electrophoresis under denaturing conditions was performed on the protease purified as described in Example VII. The results showed that the molecular weight of the major band was 38,000 and further showed that the preparation was suitable for active site titration, a technique used to estimate the specific activity of the completely purified (homogenous) protease. The active centers of the protease were titrated with N-trans-cinnamoyl-imidazole according to the method of Bender, M. L. et al., 1966, J. Amer Chem. Soc., 88:5899-5913. The specific activity of the pure protease was 62 AU/gram of protein.

The specific activity is somewhat higher than that found for the known protease, ALCALASE® (45 AU-/gram).

## EXAMPLE IX

Washing tests were performed in a Terg-O-Tometer for ten minutes at 15°C with EMPA 116 test fabric swatches, 5 cm x 5 cm (cotton soiled with blood, milk and carbon black), supplied by Eidgenossische Materialprufungs-und Versuchanstalt, Sankt Gallen, Switzerland using dosages of 0.025, 0.05 and 0.10 AU/l enzyme of Example II in the presence of Tide without phosphate (< 0.5%).

For comparison purposes, the same trials were performed at dosages of 0.025; 0.05 and 0.10 AU/l of ALCALASE®.

The cleaning ability of the proteases was measured by reflectance change ($\Delta R$) comparing the reflectance values of enzyme-washed test swatches to swatches washed without enzyme. Reflectance values were read with the aid of a Gardiner Reflectometer XL 800 (Bethesda, MD).

The results are shown in Table V.

TABLE V

| Enzyme | Dose of enzyme (AU/l) | △ R (Reflectance change) |
|---|---|---|
| | 0.025 | 5.09 |
| P. marquandii | 0.05 | 10.6 |
| | 0.10 | 13.2 |
| | 0.025 | 3.84 |
| ALCALASE® | 0.05 | 7.5 |
| | 0.10 | 11.4 |

The results indicate that Paecilomyces marquandii protease has a greater washing effect than ALCALASE® in a 15°C wash test.

**EXAMPLE X**

Based upon differences in the hydrolysis patterns for the Paecilomyces marquandii protease and Bacillus proteases (i.e., ALCALASE®), it was postulated that addition of the fungal protease to ALCALASE® would cause enhanced removal of protein stains in a wash.

Washing tests were performed as described in Example IX, except that the dosages of ALCALASE® and Paecilomyces marquandii protease were varied. ALCALASE® was either used alone (0-0.6 AU/l) or in mixtures with the Paecilomyces protease (0.4 AU/l of ALCALASE® plus 0.05-0.4 AU/l Paecilomyces protease).

The results are shown in Table VI (and Figure 3). The addition of the fungal protease does result in reflectance changes (superior wash efficacy) significantly greater than those obtained by ALCALASE® alone at the same total dosage.

## TABLE VI

Improved wash results by addition of <u>Paecilomyces marquandii</u> protease to ALCALASE® using EMPA 116 stain

| ALCALASE® dose (AU/l) | Paecilomyces marquandii protease dose (AU/l) | R (Reflectance change) |
|---|---|---|
| 0.05 | – | 7.5 |
| 0.1 | – | 10.6 |
| 0.2 | – | 13.1 |
| 0.4 | – | 14.2 |
| 0.6 | – | 16.0 |
| 0.4 | .05 | 16.6 |
| 0.4 | .1 | 18.1 |
| 0.4 | .2 | 19.5 |
| 0.4 | .4 | 20.7 |

**EXAMPLE XI**

In addition to mixing bacterial proteases and <u>Paecilomyces marquandii</u> protease at high dosages (see Example X), mixtures at lower dose rates were also tested. The dosage used in this experiment (0.1 KOU/l) more closely reflects the amount of enzyme used under actual wash conditions.

<u>Paecilomyces marquandii</u> NRRL 18048 protease was used in combination with the <u>Bacillus</u> protease, SAVINASE®, to wash blood-stained cotton. Wash tests were performed in a detergent of the following composition (grams/liter): NANSA S80S, 0.4; Berol 065, 0.15; tallow soap, 0.15; sodium tripolyphosphate, 1.75; sodium silicate, 0.4; carboxymethyl cellulose, 0.05; EDTa, 0.01; and $Na_2SO_4$, 2.1. Water hardness was adjusted to 9° dH (German hardness). Air-dried blood swatches were washed at 15°C for 10 minutes. Reflectance change was determined at 460 nm using an Elrephotometer 600.

The results with the <u>Paecilomyces</u> mixtures are shown in Table VII and Figure 4. SAVINASE®, used alone at a dose of 0.1 KOU/l, resulted in R of 2.7. <u>Paecilomyces marquandii</u>, used at the same dose rate, gave R value of 3.5. When a comparable dose of protease. consisting of a mixture of 90% SAVINASE® and 10% <u>Paecilomyces marquandii</u> was used, the R value was improved to 4.2. The observed R value of 4.2 is 50% greater than that predicted by adding the two proteases together. It is evident that, by mixing SAVINASE® and <u>Paecilomyces marquandii</u> protease, a synergistic effect is obtained. The combination of 90% SAVINASE® plus 10% <u>Paecilomyces marquandii</u> used alone in the wash experiments. Mixtures of SAVINASE® with a second bacterial protease, ALCALASE®, are shown for comparison.

TABLE VII

| Dose (KOU/l) | 0.1 (used alone) | 0.075 SAVINASE + 0.025 other | | 0.09 SAVINASE + 0.01 other | |
|---|---|---|---|---|---|
| | $\triangle$ R | $\triangle$ R* | $\triangle$ R** | $\triangle$ R* | $\triangle$ R** |
| SAVINASE® | 2.7 | – | – | – | – |
| P. marquandii | 3.5 | 3.4 | 2.9 | 4.2 | 2.8 |
| ALCALASE® | 2.2 | 1.8 | 2.6 | 1.8 | 2.7 |

\* Observed
\*\* Predicted

## Claims

1. Alkaline protease obtainable by fermentation of a protease producing strain of <u>Paecilomyces</u> <u>marquandii</u>.

2. The alkaline protease of Claim 1, characterized in that the strain is NRRL 18048.

3. Protease product characterized by comprising the alkaline protease of Claim 1 in combination with an alkaline <u>Bacillus</u> protease.

4. The protease product of Claim 3, characterized in that the alkaline <u>Bacillus</u> protease is obtainable by fermentation of <u>B. licheniformis</u>.

5. The protease product of Claim 3, characterized in that the alkaline <u>Bacillus</u> protease is of the serine type, shows optimal proteolytic activity at a pH value above about 9 and retains 80 to 100 percent of maximum proteolytic activity at pH 12, said activities being measured against hemoglobin by the Anson method.

6. A process for preparing the alkaline protease of Claim 1, characterized by cultivating a protease producing strain of <u>Paecilomyces</u> <u>marquandii</u> aerobically under submerged conditions containing suitable carbon and nitrogen sources at an alkaline pH.

7. The process of Claim 6, characterized in that the cultivation is carried out at temperatures below 35°C.

8. The process of Claim 7, characterized in that the cultivation is carried out at temperatures in the range 20-30°C.

9. Enzymatic detergent characterized by containing the alkaline protease of Claim 1.

10. Enzymatic detergent characterized by containing the protease product of Claim 3.

11. The alkaline protease product of Claim 1 in liquid concentrate form.

12. The alkaline protease product of Claim 1 in non-dusting granulate form.

13. The alkaline protease product of Claim 3 in liquid concentrate form.

14. The alkaline protease product of Claim 3 in non-dusting granulate form.

## Patentansprüche

1. Alkalische Protease, erhältlich durch Fermentation eines Protease produzierenden Stammes von <u>Paecilomyces</u> <u>marquandii</u>.

2. Alkalische Protease nach Anspruch 1, dadurch gekennzeichnet, daß der Stamm NRRL 18048 ist.

3. Proteaseprodukt, dadurch gekennzeichnet, daß es die alkalische Protease nach Anspruch 1 in Kombination mit einer alkalischen <u>Bacillus</u>-Protease umfaßt.

4. Proteaseprodukt nach Anspruch 3, dadurch gekennzeichnet, daß die alkalische <u>Bacillus</u>-Protease durch Fermentation von <u>B. licheniformis</u> erhältlich ist.

5. Proteaseprodukt nach Anspruch 1, dadurch gekennzeichnet, daß die alkalische <u>Bacillus</u>-Protease vom Serin-Typ ist, optimale proteolytische Aktivität bei einem pH-Wert oberhalb von etwa 9 zeigt und 80 bis 100 %

12

maximaler proteolytischer Aktivität bei pH 12 beibehält, wobei besagte Aktivitäten mit der Anson-Methode gegen Hämoglobin gemessen sind.

6. Verfahren zur Herstellung der alkalischen Protease nach Anspruch 1, dadurch gekennzeichnet, daß ein Protease produzierender Stamm von Paecilomyces marquandii aerob unter Submers-Bedingungen, die geeignete Kohlenstoff- und Stickstoffquellen beinhalten, bei einem alkalischen pH kultiviert wird.

7. Verfahren nach Anspruch 6, dadurch gekennzeichnet, daß die Kultivierung bei Temperaturen unter 35°C durchgeführt wird.

8. Verfahren nach Anspruch 7, dadurch gekennzeichnet, daß die Kultivierung bei Temperaturen im Bereich 20-30°C durchgeführt wird.

9. Enzymatisches Waschmittel, dadurch gekennzeichnet, daß es die alkalische Protease nach Anspruch 1 enthält.

10. Enzymatisches Waschmittel, dadurch gekennzeichnet, daß es das Proteaseprodukt nach Anspruch 3 enthält.

11. Alkalisches Proteaseprodukt nach Anspruch 1 in flüssiger Konzentratform.

12. Alkalisches Proteaseprodukt nach Anspruch 1 in nicht-staubender Granulatform.

13. Alkalisches Proteaseprodukt nach Anspruch 3 in flüssiger Konzentratform.

14. Alkalisches Proteaseprodukt nach Anspruch 3 in nicht-staubender Granulatform.


## Revendications

1. Protéase alcaline pouvant être obtenue par fermentation d'une souche productrice de protéase de Paecilomyces marquandii.

2. Protéase alcaline selon la revendication 1, caractérisée en ce que la souche est NRRL 18048.

3. Produit de protéase caractérisé en ce qu'il comprend la protéase alcaline de la revendication 1 en combinaison avec une protéase de Bacillus alcaline.

4. Produit de protéase selon la revendication 3, caractérisé en ce que la protéase de Bacillus alcaline peut être obtenue par fermentation de B. licheniformis.

5. Produit de protéase selon la revendication 3, caractérisé en ce que la protéase de Bacillus alcaline est du type sérine, présente une activité protéolytique optimale à une valeur de pH située au-dessus d'environ 9 et conserve 80 à 100 % de l'activité protéolytique maximale à pH 12, ces activités étant mesurées par rapport à l'hémoglobine par le procédé Anson.

6. Procédé pour la préparation de protéase alcaline selon la revendication 1, caractérisé par la mise en culture d'une souche productrice de protéase de Paecilimyces marquandii en aérobie dans des conditions immergées et contenant des sources de carbone et d'azote appropriées à un pH alcalin.

7. Protéase selon la revendication 6, caractérisée en ce que la mise en culture s'effectue à une température au-dessous de 35°C.

8. Procédé selon la revendication 7, caractérisé en ce que la mise en culture s'effectue à une température dans la plage de 20-30°C.

9. Détergent enzymatique caractérisé en ce qu'il contient la protéase alcaline de la revendication 1.

10. Détergent enzymatique caractérisé en ce qu'il contient le produit de protéase de la revendication 3.

11. Produit de protéase alcaline selon la revendication 1 sous forme de concentré liquide.

12. Produit de protéase alcaline selon la revendication 1 sous forme de granulés non pulvérulents.

13. Produit de protéase alcaline selon la revendication 3 sous forme de concentré liquide.

14. Produit de protéase alcaline selon la revendication 3 sous forme de granulés non pulvérulents.

FIG. IA

PAECILOMYCES
MARQUANDII

MINUTES

FIG. IB

SAVINASE

MINUTES

# FIG. 1C

ALCALASE

MINUTES

FIG. 2A

% RELATIVE ACTIVITY

+EDTA

+PMSF

TEMPERTURE

FIG. 2B

% RELATIVE ACTIVITY

+EDTA

+PMSF

pH

FIG. 3

# FIG. 4